(19) Europäisches Patentamt / European Patent Office / Office européen des brevets

(11) **EP 3 707 727 B1**

(12) **EUROPEAN PATENT SPECIFICATION**

(45) Date of publication and mention
of the grant of the patent:
**04.09.2024 Bulletin 2024/36**

(21) Application number: **18812262.6**

(22) Date of filing: **05.11.2018**

(51) International Patent Classification (IPC):
**G16H 30/40** *(2018.01)*     **G16H 50/20** *(2018.01)*
**A61B 6/00** *(2024.01)*     **A61B 6/02** *(2006.01)*
**A61B 6/50** *(2024.01)*

(52) Cooperative Patent Classification (CPC):
**G16H 30/40; A61B 6/502; A61B 6/5217;**
**G16H 50/20;** A61B 6/025

(86) International application number:
**PCT/IB2018/058663**

(87) International publication number:
**WO 2019/092575 (16.05.2019 Gazette 2019/20)**

(54) **SYSTEM AND METHOD FOR QUANTIFICATION OF TISSUE OVER TIME**

SYSTEM UND VERFAHREN ZUR QUANTIFIZIERUNG VON GEWEBE IM LAUFE DER ZEIT

SYSTÈME ET PROCÉDÉ DE QUANTIFICATION DE TISSU DANS LE TEMPS

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.11.2017  GB 201718378
20.11.2017  GB 201719206
18.04.2018  GB 201806299**

(43) Date of publication of application:
**16.09.2020  Bulletin 2020/38**

(73) Proprietor: **Volpara Health Technologies Limited
Wellington 6011 (NZ)**

(72) Inventors:
• **HIGHNAM, Ralph
Mount Victoria, Wellington 6011 (NZ)**
• **WANG, Kaier
Lower Hutt, Eastbourne 5013 (NZ)**
• **HILL, Melissa
92130 Issy les Moulineaux (FR)**

(74) Representative: **Kenny, Andrew
ipconsult
21A Commercial Road
Swanage
Dorset BH19 1DF (GB)**

(56) References cited:
**WO-A1-2014/195669     US-A1- 2010 086 182
US-B2- 8 582 840**

• **JIE-ZHI CHENG ET AL: "Automated Delineation
of Calcified Vessels in Mammography by
Tracking With Uncertainty and Graphical Linking
Techniques", IEEE TRANSACTIONS ON
MEDICAL IMAGING, IEEE SERVICE CENTER,
PISCATAWAY, NJ, US, vol. 31, no. 11, 1 November
2012 (2012-11-01), pages 2143 - 2155,
XP011491178, ISSN: 0278-0062, DOI:
10.1109/TMI.2012.2215880**

EP 3 707 727 B1

**Description**

Field of the Invention

This invention relates generally to a system and method for validating the accuracy of image parameters, especially for images used in the medical field.

Background

Breast cancer is the most common form of cancer in women over 40. The term 'breast cancer' describes several different types of disease. The majority of breast cancers are invasive ductal carcinomas (IDC), a small percentage are invasive lobular carcinomas (ILC), and a small minority are tubular carcinomas, mucinous (colloid) carcinomas, carcinomas with medullary features (related to BRCA1 gene mutation) or invasive papillary carcinomas. Rarer forms of breast cancer include inflammatory breast cancer, Paget disease of the breast and/or nipple and metaplastic breast cancer. Some forms of breast cancer are more prevalent in younger women (for example, carcinomas with medullary features, other forms are more prevalent in older women (for example colloid and invasive papillary carcinoma). The term 'breast cancer' is used here to refer to all forms of the disease.

The form and texture of tumours varies. Occasionally there is no palpable tumour and not all tumours are malignant. The cellular structure of the cancers also varies, ranging from tube-like, sheet-like or finger-like branches.

It is generally accepted that early detection of breast cancer improves patient prognosis. Breast screening entails imaging an asymptomatic population, usually using mammography, to detect breast cancers, ideally at an early stage. During screening mammography, the breast is compressed by plates using a mammography unit. Four standard images are taken during the mammographic procedure - craniocaudal (CC) and mediolateral oblique (MLO). Diagnostic mammograms are reserved for patients with breast symptoms, changes, or abnormal findings seen on their screening mammograms and patients with personal and/or family histories of breast cancer. For diagnostic mammography additional views may be taken, including geometrically magnified and spot-compressed views of the particular area of concern.

Mammograms are either 'read' once (single reading) or twice (double reading), by radiologists, radiographers and/or clinicians (referred to here jointly as 'reader' or 'readers'). Double reading, significantly improves the quality of the sensitivity of the procedure however it is labour intensive, expensive and generally subject to mandate by medical or government authorities.

X-ray penetration is an exponentially decreasing function of patient or body part thickness : a thicker breast requires a larger dose. To improve contrast, x-ray energy (kV) is decreased, however, this requires increased dose as more x-rays are absorbed.

A thicker breast would require larger dose

Furthermore, image quality is complex, with variability at each stage of a chain of steps in a method for obtaining an image of soft tissue, and interplay between each element of the imaging chain; including physical characteristics of the imaging device, image acquisition technique factors, intrinsic subject characteristics, the skill of the operator, the effect of any processing applied to the image, the method of image display, and the psychometric factors involved in image visualization and interpretation for diagnostic decision-making.

The usefulness of an image for screening purposes depends on its accuracy, and screening could be made more effective were there accurate comparison of the breast composition over time. Reliable automated means for accurate comparison would be especially effective.

US 8 582 840 B2 discloses a method for estimating the thickness of the breast during a mammogram and apply a model to compensate said thickness. This document does not disclose adjusting the compression based on the calculated thickness. This document is considered to be the closest prior art.

The phrase, 'breast composition' is used to refer to breast density and the proportion of 'dense' tissue in the breast compared to the size of the whole breast. 'Dense tissue refers to tissue which comprises fibrous and glandular tissue.

'Parameter' refers to any of a set of physical properties whose values determine the characteristics of an imaged object i.e. breast.

Dense breast tissue has been recognised as a risk factor for breast cancer : studies have estimated that an extremely dense breast represents 4-6 times an increased risk of developing breast cancer compared to an almost entirely fatty breast, and that a predominance of dense breast tissue accounts for up to 30-40% of attributable risk among average-risk population. However, the mechanism by which breast density increases breast cancer risk is unclear and the exact causality is the topic of continuous investigation , including consideration of the microenvironment local to a lesion or other region of interest.

Breast density is increasingly included in routine mammographic reports, where the prevalence (proportion) of dense tissue is determined by a reader's visual interpretation. There is an inherent degree of subjectivity in a reader assignment of breast density based on a mammogram and the rate of inter reader agreement is variable, with lowest rates of agreement reported in dense breasts and scattered fibroglandular form heterogeneously dense breasts.

Including the correct positioning of the breast: the absence of any confounding materials such as other body parts; and adequate compression pressure.

Compression of the breast is required in order to: hold the breast still (helping to prevent motion blur). immobilise the breast to avoid image non-sharpness; flatten the breast to minimise tissue overlap for better visualization; minimize the thickness of tissue that the x-rays must penetrate and the amount of scattered radiation (scatter degrades image quality) ; and reduce the required radiation dose (an average effective dose is estimated at 0.4 mSv). The breast must not be over-compressed as it may cause discomfort to the subject.

Optimally the resulting image has sufficient spatial resolution (detail) to image small structures (including micro-calcifications) and sufficient contrast to make soft tissue masses and other lesions evident.

The accuracy of the x-ray image can also be affected by a patient's anatomy and positioning, for example, the patient's height, size, weight and shape, the size and shape of the breast, the breast tissue composition, surface features and other artefacts, abnormal body habitus (such as kyphosis), and the degree of discomfort felt and tolerated by each patient.

Ascertaining the quality of an image and thereby which images are most appropriate for screening and diagnostic purposes, relies on the skill of the radiographic technologist, who might be guided by internal, or local, regional or national standards. If an image is not deemed 'acceptable' by the radiographic technologist, s/he may determine that it is necessary to acquire an additional replacement image or a variant of the original image.

Once all necessary views have been acquired and 'accepted', the images are sent to the clinician for a screening or diagnostic reading. Preferably, in preparation for the reading, other prior mammographic images from the same patient are also sent to the clinical workstations for simultaneous review. The clinician will also benefit from having related images of the breasts from other diagnostic imaging studies, both current and prior, from modalities such as, but not limited to: ultrasound; magnetic resonance imaging (MRI); positron emission mammography (PEM); breast specific gamma imaging (BSGI); and contrast enhanced spectral mammography (CESM).

The accuracy of a preliminary screening procedure is important, and comprises technical imaging parameters and physical parameters.

Recently, automated tools such as Volpara®Enterprise™ have become available which assist in appraising the quality of images. Such tools often include automated quantification of breast density, for example Volpara®Density™ according to the method described in international patent application PCT/GB2010/OO1472

However, as Wåde, Highnam et al ('Impact of errors in recorded compressed breast thickness measurements on volumetric density classification', 2016) and Ng and Lau ('Vision 20/20: Mammographic breast density and its clinical applications' 2015) have recently shown, and as Highnam and Brady previously reported ('Mammographic Image Analysis' 1999), the calculation of breast composition - specifically volumetric breast density (VBD) - although much more robust than previously thought is still prone to errors, in particular in the measured breast thickness. For example, a breast originally imaged with a thickness of 5 cm, and which does not change markedly but which is measured as a thickness of 6 cm two years later, will appear to have an increase in volume of 20% and thus a skewed density measure (using a simplified equation excluding the fatty uncompressed breast edge):

$$breast\ volume = projected\ area\ of\ breast \times breast\ thickness$$

Without verification of replicate imaging parameters such a value may not simply be skewed.

Further, breast thickness should be recorded with an accuracy of +/- 5mm, in order to ascertain the correct radiation dose for the imaging procedure. However, even wider variations are found in practice as imaging apparatus ages and/or are not adequately maintained.

Other parameters affect the accuracy of the density calculation. For example, optimally, an image of a breast will include the entire chest wall, with its fat layer and relatively lower overall breast density, compared to that of a breast imaged without the chest wall and fat layer. In practice there is significant variation in the positioning of the breast between the compression plates. Since only the portion of the breast that lies between the compression plates is imaged, such positioning errors can impact significantly on the measurements of the volumes mentioned earlier and on the estimate of breast density.

Thus with correct positioning, for example according to the method described in international patent application PCT/IB2017/054382, the projected area of the breast, the distance from the chest wall to the nipple and other image measurements can help ascertain image quality.

A further consideration is that breast composition changes over a woman's lifetime and in a dynamic and complex fashion. Rapid anatomic development at puberty and completion of differentiation at the first full-term pregnancy is followed by gradual glandular involution and structural dedifferentiation beginning in the childbearing years and accelerating at menopause. Progressive involution of parenchymal tissue with increasing age, leads to a decrease in breast density.

A further complication is that the degree and pace of change in breast composition varies greatly between women, even within the same age cohort. Glandular tissue (included here in reference to 'dense' tissue) comprises epithelial cells, which line the ductal system, and stromal elements, which provide the connective tissue framework to support the epithelium. Fatty tissue is interspersed heterogeneously between the breast lobules. In nulliparous women (women who have not given birth), lobule type 1 remains the predominant structure throughout the lifespan, while lobule type 2, present in moderate numbers during the early years, begins to decrease as early as age 23. In parous women (women who have given birth), lobule type 3 remains the predominant structure until the age of 40, after which time the breast undergoes gradual involution to lobules type 2 and 1. The regression in breast parenchyma is accelerated at menopause, where loss of endogenous estrogen and progesterone stimulates involution of glandular epithelium via apoptosis, with islands of ductal tissue left behind. There is a concurrent loss of lymphatics, and the stroma is replaced by fat.

Breast composition can also change in response to medication and/or diet. For example, breast composition may change when a woman uses hormone replacement therapy. It may also change as a woman follows a particular diet, and there is increasing evidence of impact of the metabolic syndrome on breast composition (metabolic activity has also been related to the presence of cellular atypia, which would place a patient at a higher risk for malignancy). Postmenopausal exposure to exogenous hormones has a predictable effect on tissue composition, which is dependent on interaction with estrogen receptors. Although hormone replacement with exogenous estrogen increases the mammographic density of the breast, selective estrogen receptor modulators (eg, Tamoxifen® and Raloxifene®) with antagonistic effects on estrogen receptors in the breast have been shown to reduce mammographic density. However while selective oestrogen receptor modulators and aromatase inhibitors reportedly reduce breast cancer risk, potential side effects such as propensity for other cancers, blood clots and stroke render them unsuitable for women whose density is not decreasing whilst taking such drugs.

Change in breast composition over time is becoming increasingly important both in the detection of cancers and in understanding the propensity for cancers to develop, especially amidst a drive for more monitoring and less surgery in breast cancer prevention and care.

There is also a widespread belief that a woman whose breast composition does not change over time, in particular, where the proportion of dense tissue is not reducing, may be at greater risk of developing breast cancer.

Improving the accuracy of mammography screening and diagnosis has entailed the supplementary, adjunctive use of three dimensional (3D) modalities (along with standard mammography).

'Pseudo-3D' images from tomosynthesis help with quantification, however they also display 'blur' between slices of dense tissue, require accurate measurements of breast thickness and rely on good positioning.

Background parenchymal enhancement (BPE) refers to the normal enhancement of fibroglandular tissue in an image of the breast after the administration of contrast material/agent. BPE is frequently observed in magnetic resonance image (MRI). BPE has also been reported on contrast-enhanced digital mammography (CEDM), and molecular breast imaging (MBI).

MR breast imaging provides quantitative determination of breast density via its cross-section, three dimensional coverage of the breast tissue, and high contrast between fibroglandular and fatty tissue. MRI assessment of breast density can be refined with segmentation techniques that remove the fatty tissue and quantify the amount of fibroglandular tissue. Parenchymal evaluation on MRI also benefits from its enhanced physiologic parameters as MRI allows both quantitative analysis and physiological assessment of the breast parenchyma and is affected by both the density of fibroglandular tissue and its vascularity.

BPE can affect the accuracy of interpretation and detection and has also been associated with risk of breast cancer although the association is the subject of continuing research : some studies (Hambly et al (2011) and DeMartini et al (2012)) found no increase in the incidence of breast cancer with increased BPE, and others (King et al (2011)) found a significantly increased odds ratio for breast cancer with moderate or marked BPE.

The occurrence of BPE has also been associated with greater sensitivity to the effects of physiological parameters and chemo-preventive therapies aimed at blocking breast cell proliferation.

Visual breast tissue assessment protocols, such the American College of Radiology Breast Imaging Reporting and Data Systems (BI-RADSO) 5th Edition, classify BPE as 'minimal' (less than 25% glandular tissue demonstrating enhancement), 'mild' (25% - 50% glandular tissue demonstrating enhancement), 'moderate' (50% - 75% glandular tissue demonstrating enhancement) or 'marked' (more than 75% glandular tissue demonstrating enhancement).

Several studies have suggested that BPE further represents physiological hormonal enhancement, reflecting hormone-related changes in breast composition and vascularity : for example, fluctuations in BPE have been demonstrated throughout the menstrual cycle (with the highest levels of enhancement in the second half of the menstrual cycle during the luteal phase when breast cell proliferation is at its highest). BPE has also been demonstrated to reflect variations in oestrogen-mediated vascular permeability, with increased BPE seen in women taking oestrogen replacement therapy, and decreased BPE with anti-oestrogen medications and in postmenopausal patients.

However, the specificity of breast MRI is variable, and its efficacy where there is high BPE is not known. MRI is expensive - an estimated ten times the cost of mammography. Further, MRI is associated with a significant 'false positive' rate in contrast to mammography which has a tolerable rate of false-positive recall, and is increasingly subject to quality measures and improved quality assurance.

Means to analyse an x-ray to determine breast density and to predict BPE would both indicate whether MRI were likely to be effective (e.g. appropriate, cost effective and 'patient friendly'), whether other adjunctive modalities would be more suitable and provide important means to evaluate a patient's risk of breast cancer, for example, as a feature integral to a breast cancer risk model.

Means to predict BPE based on quantitative analysis of a mammogram-derived tissue composition map and use of BPE as a predictive parameter of image quality and risk of disease would be of value.

Computer aided detection (CAD) is a process whereby pattern recognition software identifies and marks suspicious features on an image in order to bring the suspicious features to the attention of the reader; or to assist the reader once they have identified a suspicious feature. For example, a reader may first review an image without CAD, then activate the CAD software and re-evaluate the CAD-marked areas before issuing their observations and final report.

CAD also has the potential to improve workflow efficiencies by increasing the detection of disease and reducing the false negative rate for example due to visual oversight. The use of a computer rather than a second human observer has the advantage of not increasing the demands on the reader or clinical resources, without undo impact on the recall and work up rates.

In mammography CAD algorithms search images in digital format, such as images acquired via full field digital mammography (FFDM) and tomsynthesis for features such as microcalcifications and masses, spiculated and non-spiculated, architectural distortions and asymmetries either via processing a 2D mammogram, the central projection (or more) of a 3D tomosynthesis sweep, or a slice (or slices) from a 3D tomosynthesis reconstruction.

However, in practice CAD systems do not mark all actionable findings and the absence of a CAD mark on a feature or region of interest (ROI) of concern to the reader from their pre-CAD review may deter further evaluation. In this instance, the false negative report would be the result of an interpretive rather than a visual perception error.

Furthermore, CAD algorithms mark features that meet the algorithm requirements and not only those features that the reader considers to warrant further investigation, i.e. false CAD marks.

Thus in practice CAD generates many more false CAD marks than true CAD marks and it remains the responsibility of the reader to determine if a CAD mark warrants further evaluation.

Several methods have been devised to reduce the incidence of false positives. Often such methods provide means to sort the CAD marks according to predetermined criteria. For example, the marks may be displayed temporally, and/or in sequence within an image data set in accordance with a generated list; and/or displayed with an indication of their respective position on the list. Many methods allow display criteria to be added to the list on-going.

Other methods to reduce the incidence of false positives entail interactively displaying the CAD results along with an indication of likelihood of abnormality in the imaged tissue. Based on tissue location the marked object is selectively related to statistical occurrence/probability of abnormality and the rating is converted into a probability measure. For example, an image e.g an x-ray mammogram image of a breast in digital form, is received and processed by computer to generate an altered or second version that differs by image shift, image rotation, and image inversion. Each version is individually processed using a foundational CAD algorithm to generate a respective individual CAD detection set. The CAD detection sets are then compared to generate an overall CAD detection set, thus reducing the false positive rate.

In a clinical setting, CAD is based around collating an in-house database and perfecting the algorithm in- house, often complemented by reader studies. The technology is applied to and reported on single images. The report, typically within a digital imaging and communications in medicine (DICOM) CAD Structured Report (CAD SR), contains potential marks, and higher probability marks.

Thus CAD solutions incorporate probability of relevance, workflow efficiencies and other parameters such as size and colour of marked features.

However, the clinical utility of CAD is far from clear. According to some reports, so many false positives are generated - including on optimally compressed, clear images - that readers simply ignore all the marks. As described above, interactive methods for reducing false positives provide technical means to correct this, but ultimately rely on the reader to search for potential marks before seeing any results, which can itself be frustrating for the reader and counterproductive.

Furthermore, parameters such as the significance of the distribution of CAD marks is not currently determined, although CAD marks in one part of an image and not another might indicate technical imaging parameters such as blur or poor compression rather than features of interest e.g. cancers.

It is an advantage of the present invention that it can be applied to CAD to determine the efficacy of an image parameter in order to improve the accuracy of the CAD marker(s) and enable an accurate comparison over time to assess a change in the composition of the imaged object over time; and to inform a (diagnostic) risk model.

Summary of the Invention

According to a first aspect of the invention there is method for validating one or more native image parameters from one or more images of a source object, wherein: one or more native parameter(s) from the image(s) is analysed with a reference data to determine whether the native parameter(s) is/are plausible, characterised by transforming quantitatively the image(s) to a tissue composition map(s).. This may be the first step (STEP 1) or start of the first step in the method.

According to a second aspect of the invention there is a system for implementing the method of the invention. The system includes an apparatus for validating the native image parameters, including: a device to utilize the one or more images of the source object, a device to analyse the one or more native parameters with the reference data to determine whether the native parameters are plausible, and a device to transform quantitatively the image(s) to the tissue composition maps.

Advantageously the accuracy of native image parameters as indicators of image quality and/or data integrity is improved.

The reference data may include statistical information which characterises the image.

The image may be a source image. The image may have a raw or processed format. The image may be a derived image which is derived from the source image. An example of a derived image is a map of segmented regions or a tissue composition map. Other types of derived images are also applicable.

The native parameter(s) may be obtained via values from the tissue composition map(s) derived from the source image(s).

The reference data may includes information derived from the image which characterises the source object. The reference data may include information determined by observation of the source object.

One or more of the native parameters may be directly extracted from the image. Preferably the native parameter(s) which is/are directly extracted includes pixel values from the source image.

A native parameter for which plausibility is determined is preferably breast thickness, which is the thickness of the breast when it is compressed when the image is obtained. A compressed breast thickness that is plausible may be obtained from the image via pixel values of the image breast. The compressed breast thickness so obtained may be indicated to user as the 'true' breast thickness. The 'true' breast thickness may be used in further calculations and/or correlations to determine plausibility of other native parameters.

Native parameter(s) which is/are directly extracted may include information recorded into the image including patient age, compressed breast thickness, compression device type, compression force, applied image processing, or presence of an implant.

Preferably one or more of the native parameter(s) is/are directly compared to the reference data. Preferably the native parameter(s) are compared directly to the reference data.

The reference data may be internal (i.e. from the same source image). The reference data may be external (i.e. from other images or from statistics derived from the image or source object). The reference data may be both internal and external.

Preferably additional source images of the source object are used. Advantageously the accuracy of the native image parameters as indicators of image quality and/or integrity is improved from the source object in the source image. The quality and/or integrity may be improved for the same source object in the additional source images also.

Preferably the native parameters are derived from one or more regions of the image(s).

The reference data may be derived from the same image as one or more of the native parameter(s).

At least one of the native image parameters for each of the images may be used to ensure that the same part of the source object is being compared. Preferably the projected area of the breast and chest wall to nipple distance are used in a plurality of the images to ensure that the same part of the breast is being compared.

The reference data and the native parameter(s) may be derived from a single individual image which may be a source image.

The reference data may be obtained otherwise than from the same image as one or more of the native parameter(s). One or more of the native parameters may be obtained from an additional image.

The image or some of the images may obtained at substantially the same time. The images or some of the images may be obtained at substantially different times. Advantageously the accuracy of the image parameters is improved for the same source object acquired in the image and additional images over time.

The method may have a second step (Step 2) which preferably follows the first step. The second step may have two parallel parts (Step 2A) and (Step 2A). The two parallel parts may be carried out independently. The method may include Step 2A without Step 2B and vice versa.

Preferably the native parameter(s) is obtained via pixel values from a source image(s) having a raw or processed format or obtained via values from the tissue composition map(s) derived from the source image(s).

In the second step, and preferably part Step 2A, of the method, plausibility of one or more of the native parameters is determined individually with respect to the reference data. The native parameter may be breast thickness which is measured, estimated, and/or calculated based on a first image or other selected image which may be a source image. The first or selected image may then be used as a baseline image.

In step 2A, the native parameter for which plausibility is determined may be breast thickness obtained from the image or source image via pixel values of the imaged breast. Preferably this step includes measuring and/or estimating breast thickness based on a baseline first source image.

Preferably the breast thickness is measured and/or estimated based on values from the tissue composition map(s) of the image and utilizing the tissue composition map as a base image.

Preferably this also includes resolving or compensating for error in the breast thickness based on constant volume or clinical observation.

In the second step, and preferably part Step 2B, plausibility of all of the native parameters in a collective group is determined. The plausibility of one or more image native parameters may be estimated from a collection of images In this way, non plausible images may be identified. The collection of images may include different views of the same source object.

The plausibility of the parameters within the collective group may be determined with respect to the reference data. The plausibility of the parameters within the collective group may be determined by a relationship of native parameters in the collective group.

The collective group native parameters may be breast thickness of an imaged breast in a plurality of images. Preferably the plausibility of any of the breast thickness in the group is estimated according to a comparison with the breast thickness in source images of the group having another view of the imaged breast. Some images may be CC views and some images may be MLO views and some images may have another differing views. The breast thicknesses the image breast in these differing views may be compared.

A non-plausible native parameter which relies on one or more variable features may be modified. A single step or an iterative method may be used to modify the native parameter.

Preferably an adjusted image parameter is calculated for any native parameter(s) determined to be non-plausible which relies on a variable feature of the image(s) or source image(s) or derived map(s) by adjusting the native parameter(s) towards plausibility.

The adjusted image parameter may be calculated after the first step.

The adjusted image parameter may calculated following determination in the second step of non plausible native image parameters. The adjusted image parameters may calculated following detemination of non plausible image parameters in Step 2A or Step 2B or both. In this situation the adjusted image parameter maybe calculated in a step denoted Step 3. Step 3 is not however necessarily performed third.

A variable feature may be compressed thickness and/or breast volume.

The native parameter(s) which relies on the variable feature may be compressed breast thickness or breast volume or both.

Preferably the native parameter(s) is/are adjusted toward plausibility based on specific characteristics of to the source object.

Preferably information utilised to determine the quantity and direction of adjustment includes density breast density or breast position. Preferably the breast density is obtained from first image or other selected or baseline image. Breast density may be determined from a map of segmented regions and/or a tissue composition map. Breast position may derived from measurements of the source object (i.e. the breast) an image or from clinical observation or from information in the DICOM header of an image.

Preferably native parameter(s) is/are adjusted toward plausibility based on technical image characteristics. The technical image characteristics may include paddle type which compressed the breast when the source image was obtained or image processing method of the source image.

The plausibility of the adjusted image parameter(s) is/are assessed and the native parameter(s) may be adjusted again iteratively to recalculate the adjusted image parameters.

An error threshold may be preselected a native image parameter based on the information specific to the source object or the technical characteristics. Above a preselected error threshold, the method may include adjusting compression based on breast thickness.

Preferably compression is adjusted based on adjusted breast thickness.

A value of compression associated with a breast thickness from one or more images may be adjusted based on the information specific to the source object or the technical characteristics. The value of compression may also be adjusted from reported or recorded clinical observation. A value of breast thickness of the breast may also be determined. The breast thickness may be recalculated by adjusting a breast thickness utilizing the value of compression.

Compressed breast thickness maybe the native image parameter determined to be non-plausible in at least one of a plurality of images obtained at different times. The compressed breast thickness may be adjusted toward plausibility by a computation in which constant breast volume is assumed.

An average breast volume may be used to calculate a target breast volume at the time each source image is obtained, and the breast thickness is adjusted to achieve that. A breast volume reported or recorded as the image is obtained may be compared to the target breast volume to determine whether the reported breast volume and/or the adjusted breast thickness are plausible.

There are some non-plausible parameters which cannot be modified. For example a non plausible parameter may not be modified because it relies on an integral property or characteristic of the image. The accuracy of the non plausible parameter may be estimated and preferably weighted based on the estimate of accuracy. A confidence or confidence level may be estimated for the non plausible parameter. The confidence may be estimated with reference to the context of the parameter.

A step of assigning parameter confidence level/weighting may occur in parallel with the step wherein an adjusted image parameter is calculated for any native parameter(s) determined to be non-plausible. The step of assigning an adjusted image parameter may be calculated for any native parameter(s) determined to be non-plausible may follow directly after the first step or it may follow the second step, step 2A or step 2B.

The step of assigning parameter confidence level/weighting may occur in parallel with the step of adjusting non-plausible parameters towards plausibility, i.e step 3.. Consequently the step of assigning parameter confidence level/weighting may be designated step 4 even though it does not necessarily occur fourth.

In a first part of the step of assigning parameter confidence level/weighting, step 4A, a weighted image parameter may be calculated by assigning a weighting to any native parameter(s) determined to be non-plausible which relies on an integral property of the source object. The integral property may be a foreign object, presence of cancer, predicted BPE class or breast arterial calcification.

The weighting may be determined by a preselected correlation between confidence level in the native parameter determined to be non-plausible and the predicted BPE class.

Based on a regression model, predicted BPE measures derived from breast tissue composition maps may be categorised into ordinal BPE classes. Preferably the breast composition maps are determined from processing a mammogram source image to generate a density map as a standardized base image.

Images of the source object obtained at different times may be processed so that accuracy of temporal BPE prediction is improved. Preferably BPE class is determined from processing a mammogram source image to generate a density map as a standardized base image.

A series or collection of images obtained at different times may be processed to obtain a BPE class over time. The BPE classes obtained over time may be compared or related with a preselected correlation, algorithm or model of BPE class over time to improve accuracy of temporal BPE prediction.

Texture features may be extracted from a region of interest in the density map and used in the regression model. the regression model may be a proportional odds model to calculate the possibility of the texture features falling into any one ordinal BPE class.

Fractal dimension of a tissue pattern in a breast tissue composition map may be used to derive BPE category classes correlated complexity of the structure of the breast tissue.

A weighted image parameter may be calculated by assigning a weighting to any native parameter(s) determined to be non-plausible which relies on a fundamental image characteristic. This may be a variation of Step 4 or it may be an additional part step 4B of step 4.

Fundamental image characteristics are imparted to the image by physical properties or limitations of devices used to obtain the image, and by principles of physics which govern acquisition of the image. A fundamental image characteristic may be motion blur, image noise or image contrast.

Preferably image parameters are checked for whether they are used in multivariate measure. If the image parameters are used in multivariate measure, then the multivariate measure may be re-generated using at least one adjusted image parameter. Image parameters and/or image maps may be determined using an algorithm or model with multiple input measures.

Preferably input measures for multivariate measure include volumetric breast density, predicted BPE category, change in breast density over time, patient dose, breast arterial calcification measures or scores, CAD markers, and/or risk of diseases.

Step 3 may occur in parallel with step 4. Step 2A and step 2B may also occur in parallel with step 3 and step 4.

In another step a method at least one of the image parameters used in multivariate measure is re-generated using at least one adjusted image parameter. This step may be denoted step 5. Step 5 may follow after step 1, 2A, 2B, 3, 4A or 4B.

In step 5, image parameters and image maps determined using algorithms of models with multiple input measures may be recalculated or regenerated using the adjusted or weighted image parameter from step 3 and/or step 4.

At least one of the image parameters used in multivariate measure may be regenerated using at least one weighted image parameter.

Changes in composition of the source object over time may be calculated to inform a risk model.

Computer aided detection is applied to the image(s) using pattern recognition to identify and CAD mark features on an image. The mark features may be placed on a source image or an image derived from the source image to assist a person searching for features of interest in the source object.

The CAD marked features may combined with parameters determined to be plausible to ascertain an overall risk score.

The overall risk score may comprise a comparison of discrepancy between quantity and types of CAD marked features. The overall risk score may comprise a comparison of discrepancy between CAD marked features on contemporaneous images.

Preferably image parameters and outputs of models and/or algorithms which utilize the image parameters are output to users for interpretation and application. The image parameters and the outputs of the models and/or algorithms may also be stored for future use. Remote electronic output and storage means may be used.

The present invention relates to a system and method for validating the accuracy of image parameters.

Advantageously the method for validating one or more native image parameters may be used to determine the accuracy of the image parameter(s). Preferably least one parameter from the image, such as an x-ray image of a breast, and at least one aspect of an image, for example, the position of a part of the imaged object, is used to determine the accuracy of the image parameter.

A step (a) may include ensuring that the same part of the images object is being compared. Preferably in step (a) the part of the image is compared by superimposing corresponding areas on to each other using image registration techniques.

A step (b) may include measuring/estimating breast thickness based on a baseline correct image. Preferably in step (b) a baseline correct image is generated using a constant breast volume. The baseline correct image may be calculated via average over multiple images.

A step (c) may include resolving or compensating for errors in breast thickness (based on volume e.g. a constant volume, and/or clinical observation). Tomosynthesis projections from different angles may be used to improve breast thickness estimation.

A step (d) may include above an error threshold, adjusting compression based on thickness.

A step (e) may include determining a value of thickness of the breast.

A step (f) may include calculating changes in the composition of the imaged object over time to inform a risk model.

BPE category/class may calculated. Preferably BPE category/class is calculated using fractal dimension. Preferably to accurately and reliably determine BPE, feature selection is performed to reduce the dimension and the complexity of the modelling using one of backward selection, learning vector quantization model, recursive feature elimination, Boruta algorithm and least absolute shrinkage and selection operator.

According to an aspect of the invention there is a method for validating a native parameter from an image of a source object, wherein: one or more native parameters from the image is analysed with a reference data to determine whether the native parameter(s) is/are plausible. Preferably the image is a source image or a derived image.

Hence, according to the present invention there is a method using one or more parameter(s) of an imaged source object in a source image, comparing the parameter(s) to known, statistically inferred and/or observational object characteristics to determine the efficacy of the image parameter(s), and to subsequently adjusting one or more of the parameters which have efficacy less than a preselected limit according to their relationship to the source object. Preferably the method includes determining whether the relationship is plausible., Preferably the method includes similarly assessing the efficacy of image parameters derived from two or more source or derived images of the same source object acquired in different orientations, or at different points in time, for their collective evaluation, whereby parameter efficacy is determined according to comparative analysis., One or more parameters with limited efficacy may be adjusted according to a plausible comparative relationship to the source object.

Advantageously the method to determine the accuracy of an image parameter. Preferably at least one parameter from the image, such as an x-ray image of a breast, and at least one aspect of an image, for example, the position of a part of the imaged object, is used to determine the accuracy of the image parameter.

According to an aspect of the invention there is a method for validating accuracy of native parameters of an image, including: obtaining one or more images of a source object, analysing the images to obtain native parameter(s) of the source object, and analysing the native parameters in conjunction with reference data to determine whether the native parameter(s) are plausible individually or plausible collectively.

When more than one image of a source is obtained, preferably the images are all obtained at substantially the same time, for example or one the same day in the same week.

Alternatively, when more than one image of a source image is obtained, each of the images may be obtained at a substantially different times, for example a month or a year or more apart.

In situations where one or more of the native parameters are not plausible individually, then preferably the method includes determining whether the not the individually non-plausible native parameters are modifiable.

In situations wherein at least some of the image parameters are not plausible collectively, then preferably the method includes determining whether the not collectively plausible native parameters are modifiable.

Preferably before determining whether any of the not plausible parameters are modifiable, the method includes determining whether the native parameters are plausible individually or whether the native parameters are determinable collectively.

Preferably the step of adjusting non-plausible parameters toward plausibility is executed in parallel with the step of assigning parameter confidence and/or weighting.

Preferably the method includes determining whether the native parameters are used in multivariate measure and if they are, calculating individual and collective measures before storing results and providing output results to a user.

Preferably the step of determining whether the native parameters are used in multivariate measure follows after the step of adjusting non-plausible parameters toward plausibility. Preferably the step of determining whether the native parameters are used in multivariate measure follows after the step of assigning native parameter confidence level or weighting.

Preferably the step of adjusting non-plausible parameters toward plausibility follow after, preferably immediately after, determining at least some of the non-plausible parameters are modifiable.

Preferably the step of determining whether native parameter(s) is/are used in multivariate measure follows after, preferably immediately after determination that at least some of the native parameters are plausible individually or plausible collectively.

Preferably the step of adjusting the non-plausible parameters toward plausibility and/or the step of assigning the parameter confidence level/weighting occur intermediate the steps of determining whether the non-plausible parameters are modifiable and the step of determining whether the parameters are used in multivariate measure.

Consequently all the native parameters obtained by analysing the images are checked to determine whether they are used in multivariate measure, although the native parameters which are found to be not plausible are first either adjusted toward plausibility or assigned a confidence level/weighting.

Preferably the method for validating accuracy of native parameters of an image, including: obtaining one or more images of a source object, analysing the images to obtain native parameter(s) of the source object, and analysing the native parameters in conjunction with reference date to determine whether the native parameter(s) are plausible individually or plausible collectively, then checking whether the native parameters are plausible individually and/or checking whether the native parameters are plausible collectively, then checking whether native parameters determined to be not plausible are modifiable and adjusting the parameters that are modifiable towards plausibility and assigning confidence or weighting levels to parameters that are not modifiable, checking all the obtained native parameters of the source object to determine whether any are used in multivariate measure and calculating individual and collective measures of those that are used in multivariate measure, and then storing and outputting information determined during the course of the performing the method.

In particular, this invention uses parameters to classify tissue and tissue features of an imaged object in order to assess change in the composition of the imaged object over time and to inform a (diagnostic) risk model. Thus, it is an advantage of the present method that improvements to image parameter efficacy can increase accuracy of the estimation of the composition of an imaged object, and especially to a change in the composition of an imaged object over time, subsequently improving (diagnostic) risk estimation.

The invention will now be described, by way of example only, with reference to the accompanying drawings in which:

Brief Description of the Figures

Figure 1 shows a method of image parameter accuracy assessment and improvement;

Figure 2 shows a difference between the projected area in an image at an earlier time 'Time T' and a later time 'Time T+1'.

Figure 3 shows an image at the later time 'T+1' with the same projected area as at the earlier time 'Time T', but with a different reported breast thickness.

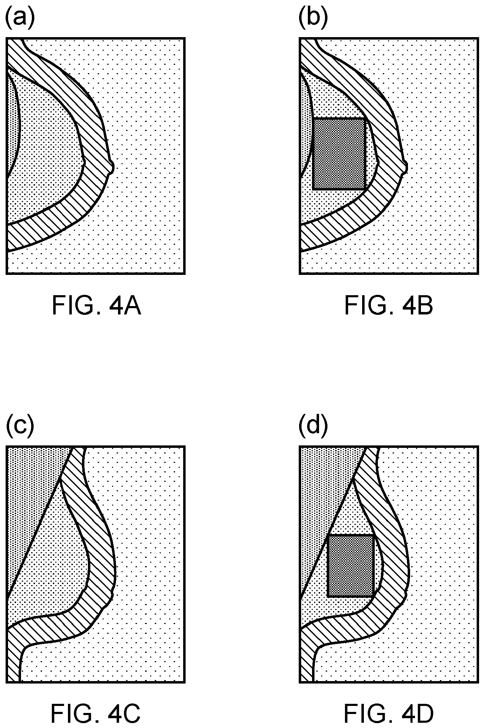

Figure 4 shows a region of interest as a maximised rectangle in the inner breast for CC (a and b) and MLO (c and d).

Figure 5 shows confusion matrix for the target (truth) and output (prediction) classes. The BPE categories are predicted using all texture features listed in Table 1.

Figure 6 shows an example of extracting fractal dimensions from different binary images.

Figure 7 shows a confusion matrix for BPE prediction from fractal dimension. The overall accuracy is 70.3%, moderately dropped from 94.6% in Fig. 5 where all texture features are used.

Figure 8 shows a confusion matrix for BPE prediction from VBD only. The overall accuracy is 37.8%, compared to 70.3% using fractal dimension in Fig. 5 and 94.6% using completed set of texture 5 features in Fig. 5.

Figure 9: shows an example of two breasts with distinct VBD and texture characteristics: the left breast is very dense but its BPE category is minimal; the right breast is fatty but its BPE reading is moderate.

Figure 10 shows a learning vector quantization reported feature rank by importance

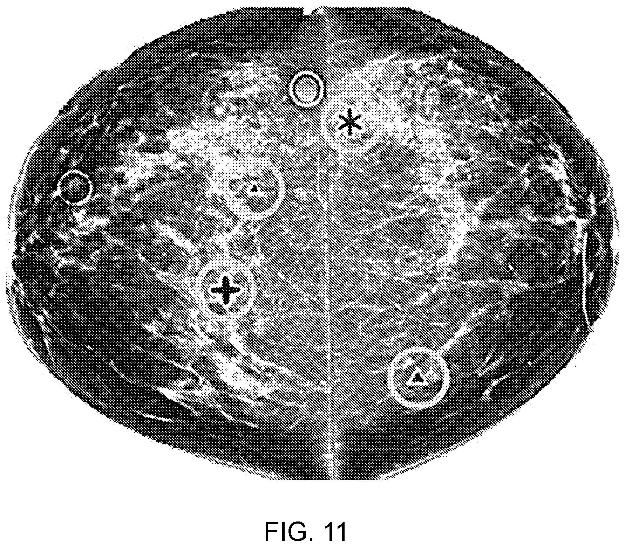

Figure 11 shows an example where the CAD system has marked up a set of suspect areas which the radiologist should potentially take a second look at.

Detailed Description of the Invention

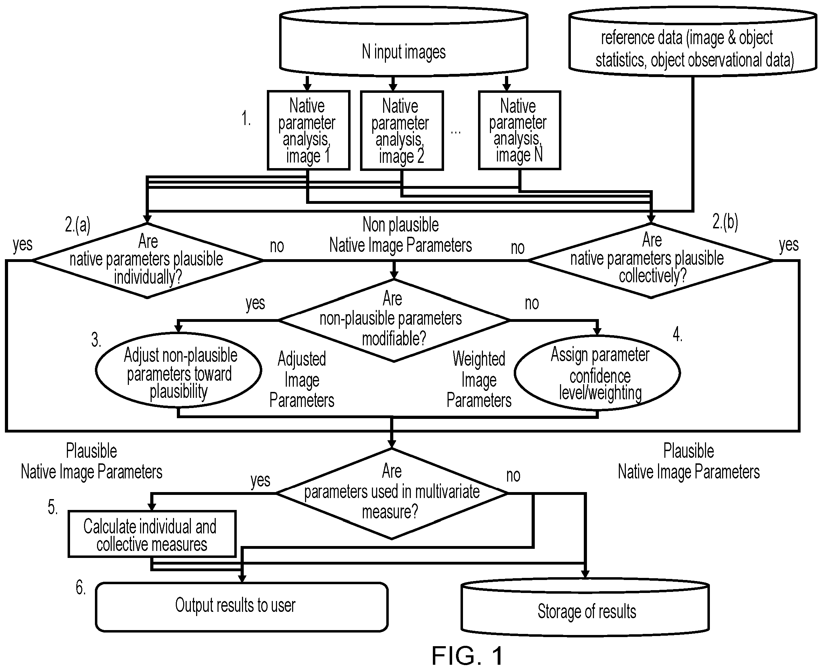

In an illustrative embodiment in Figure 1, the method for image parameter validation includes the following key steps.

STEP 1:

At least one parameter is derived from each of one or more images of the imaged source object, i.e. x-ray images of a breast in mammograms. The at least one parameter is derived from one or more regions from each image, i.e. the x-ray image of the breast. These images are herein referred to as 'source' images.
Parameters derived from a source image are herein referred to as 'native' parameters. For example, native parameters from a mammogram may include aspects of the image, or image features that can be directly extracted. Directly extractable native parameters include image pixel values and information from the DICOM header of the mammogram. Information in the DICOM header usually includes patient age, compressed breast thickness, compression device type, compression force, applied image processing, presence of an implant, anatomical view (e.g., CC, MLO), and acquisition technique factors (e.g., kVp, mAs, anode/filter combination).
Native parameters may also rely on indirect measurement, or estimation using one or more methods and algorithms. Examples of indirectly measured native parameters include: tissue composition, breast volume, texture descriptors, measures of image contrast and noise, the presence and/or location of any foreign objects in the image (e.g., other body parts, biopsy clips, scar markers, etc.), detection of motion blur, measurement and scoring of breast positioning, prediction of BPE, and the detection, classification and scoring related to lesions that may include cancers, benign findings, and arterial calcifications.
A radiographic image may be transformed quantitatively to a tissue composition map indicating a total amount of organ tissue. A calcification map may be generated indicating position in the tissue composition map of calcified tissue. A calcification free tissue composition map may be generated from the tissue composition map using the position of calcified tissue in the calcification map. A vessel map of the position of vessels in the tissue composition map may be generated. The vessel map may be combined with the calcification map to generate a map of vessel calcification indicating the position of calcified vessels in the tissue composition map.
The tissue composition map comprises quantitative values of total amount of organ tissue associated with respective quantitative values of position in the map. A quantification measure of vessel calcification of the organ is generated from the vessel map. The location and/or quantity of vessel calcification may be used for disease risk prediction and stratification.
The calcification density and/or mass may thereby be measured using tissue composition information from the tissue composition map combined with a vessel map generated using a segmentation algorithm.

STEP 2:

In a second step, the native parameters from an individual source image are assessed for their plausibility according to how they compare to reference data. Herein the term plausible is used to refer to the native parameter accuracy.

Some of the reference data may be internal, where a given native parameter is compared to one or more native parameters from the same image. In an internal comparison the given native parameter is compared to one or more other native parameters from the same image.

Other reference data is external, where a given native image parameter is compared to one or more native parameters obtained otherwise than from the same image. For example, the given native parameter may be compared to previously measured image and object characteristics, for example, in a statistical manner, to reference appropriate constraints. Also, observational data, such as notes collected from a technologist on patient body habitus, general health status, previous findings, and/or family history can be applied to determine parameter plausibility.

Both internal and external data may be used to derive one or more reference values against which the parameter plausibility is assessed.

Two types of estimates of plausibility are made. The first type of estimate for whether a given parameter is plausible is assessed on an individual-image basis (Step 2a). The second type is assessed using a collection of images. (Step 2b).

STEP 2A

In an example of the first type of estimate (Step 2a), a plausible breast compressed thickness is estimated according to internal comparisons via expected imaged object pixel values given the applied imaging technique factors.

STEP 2B

Then in the second estimate (Step 2b) the plausible breast thickness is estimated according to comparisons with a collection of compressed thicknesses determined from other views of the imaged object.

STEP 3:

In a third step, non-plausible native image parameters that have a value or score that can be feasibly modified because the parameter relies on one or more variable features of the image of the imaged source object (e.g., compressed breast thickness, breast volume), are adjusted towards plausible values based on the imaged source object (e.g., density on most plausible image, breast position) and technical characteristics (e.g., paddle type & image processing employed). This may be done in either a single step, or as an iterative process. In the iterative process small adjustments are made to an image parameter, the plausibility of this adjusted parameter is assessed, and further adjustments may be made if the parameter continues to be non-plausible. Herein, an adjusted image parameter refers to an image parameter whose native value has been modified.

STEP 4:

In a fourth step, non-plausible native image parameters that have a value or score that cannot be feasibly modified because they rely on an integral property of the imaged source object (e.g. foreign object, presence of cancer, predicted BPE category, and breast arterial calcification), or a fundamental image characteristic (e.g., motion blur, image noise, and image contrast), are assigned weights, or a parameter confidence level that can be used to estimate the relative accuracy or efficacy of each of the native image parameters. For example, the confidence in the presence of cancer, or breast arterial calcification in an image with a large amount of motion blur may be low. Similarly, the confidence in the detection of motion blur may be reduced if there is a large foreign object, such as an implant present, which obscures much of the breast tissue.

STEP 5:

In a fifth step, image parameters that are determined using algorithms or models with multiple input measures [e.g., VBD, predicted BPE category, change in density over time, patient dose, CAD markers, risk of disease(s)] are re-calculated or re-generated using the adjusted or weighted image parameters with the intent that the result has superior accuracy compared to the use of native image parameters.

STEP 6:

In a sixth step, the image parameters and the outputs of models and algorithms dependent on their use are output to users for their interpretation and application, and also stored for their future use.

An advantageous application of the above method is to first obtain accurate temporal data by ensuring that the same part of the imaged object is being compared in different source images. For example, to ensure that the same part of the imaged object is being compared, in mammography, the projected area of the breast is used, along with a parameter such as chest wall to nipple distance, and/or a selected an area proximal to a feature, for example, an area of 1-5 cm2 closest to the nipple.

One method to ensure that the same part of the imaged object is being compared comprises superimposing corresponding areas on to each other using image registration techniques (any one of a number of deformable image registration methods known to those versed in the art).

Far more difficult is how to resolve or compensate for errors in breast thickness in each source image. In an embodiment, certain assumptions are made about a woman's breast characteristics over time, such as that over a one-year period, the breast volume should stay the same, or breast volume has changed due to weight gain or weight loss.

According to the present invention, variations in a woman's breast characteristics over time can be deduced from major changes in breast volume (i.e. beyond what errors would produce) and/or by having clinical observations input to the algorithm (e.g. 'Woman lost x number of lbs/kg'), or via user input in some manner ('Bra size changed from x to y.').

Given the deduction of variations of the woman's breast characteristics over time, new metrics are computed in order to correct the breast thickness. For example: if the projected area at Year 2 is the same, or similar to Year 1, then it is assumed that the breast size has not changed markedly. So, the breast thicknesses should therefore be the same to get the same overall breast volume. Alternatively, a change in the projected area(s) of for example +/- 10%, might indicate the need for more or less breast compression, in order to adjust the breast thickness, whether larger or smaller, to get to the same overall breast volume.

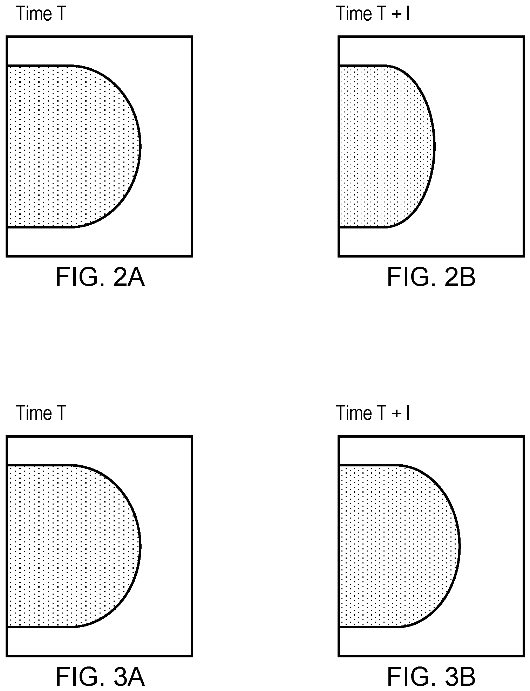

With reference to the figures 2 and 3 examples are shown for how to resolve or compensate for an error in breast thickness in one or the other source image., in Figure 2 a source object in a source mage at earlier time T has considerably different projected area than the source object in a source image at later time T+1. Using the source images themselves, we can work out that this difference is likely to be due to different positioning of the breast rather than a change in breast volume.

In Figure 3, the source object in the source image at earlier time T has the same projected area as at later time T+1. However, in this example the compressed breast thickness extracted as a native parameter from the DICOM header of the source image at earlier time T is different than the compressed breast thickness extracted from the DICOM header of the source image at later time T+1. So, the reported breast thicknesses are very different. As the projected areas are the same, we deduce that one of the breast thicknesses might be incorrect and use assumptions, such as constant breast volume, to correct the breast thickness. A baseline 'correct' image in thereby provided. Thus, the compressed breast thickness in one of the source images is deemed non-plausible and then adjusted toward plausibility by a computation in which constant breast volume is assumed.

In a further embodiment an average of a native parameter might be calculated over multiple images to establish the baseline correct image. For example, the average of the breast volume over 5 years is used to calculate a target breast volume at each year, and the breast thickness(es) adjusted to achieve that.

Similarly, the same breast thickness for all 5 years might be used. While some 'absolute' density might be lost, maintaining a uniform thickness might yield a more accurate 'change in density'.

Thus, in the present invention the native parameters are used as a means of internal checks on each other. The relationships are complex, with, for example, breast thickness impacting not just on breast volume but also on contact area, and on where certain reference points are found. The present invention thus comprises a means whereby these internal checks identify 'suspect' values, enabling either user intervention or automated correction/compensation.

In tomosynthesis, where multiple projections from different angles are taken it might be possible to use the views of the breast in those different projections to improve breast thickness estimation.

It is a further advantage that the parameters include a predictive BPE category.

Based on a regression model, predicted BPE measures derived from breast tissue composition maps are categorised into four ordinal BPE classes: Minimal < Mild < Moderate < Marked.

In a method for determining BPE class, a raw mammogram is processed and a density map (density map refers to a graphical representation where the thickness of dense tissue is mapped at each pixel intensity and displayed as a height surface, the height corresponding at each pixel (x, y) to the thicknesses of dense tissue at that location for a quantitative representation) is generated as a standardised base image.

A maximised area in a region of interest, for example a rectangular area ('maximised rectangular area' is used here to describe the largest rectangular region of interest ('ROI') which fits inside the inner breast area) is then selected from the inner breast using a segmentation map (Figure 3) to isolate a ROI. Texture features are extracted from the ROI in the density map. The texture features comprise traditional measures such as mean, variance, skewness and kurtosis. Factoral feature vectors are constructed as described in Costa et al 2012 using single- and multi-level Otsu algorithms to generate a series of binary mask images, each of which further yields three features: valid pixel count (mask area), mean grey level and fractal information.

The texture features are then used for regression of a multinomial ordinal logistic model. Multinomial ordinal logistic regression comprises a matrix constructed out of mammographic images where each row corresponds to a feature: for example, 37 mammographic images are used to construct a 37×20 feature matrix, where each row corresponds to the 20 features from an image. A regression model is described by a proportional odds model in equation (1) :

(1)

$$\ln\left(\frac{P(\text{Class} = i)}{P(\text{Class} \neq i)}\right)$$
$$= \alpha_0^{(i)} + \alpha_{\text{mean}}^{(i)} X_{\text{mean}} + \alpha_{\text{var}}^{(i)} X_{\text{var}} + \alpha_{\text{skewness}}^{(i)} X_{\text{skewness}}$$
$$+ \alpha_{\text{kurtosis}}^{(i)} X_{\text{kurtosis}} + \alpha_{\text{entropy}}^{(i)} X_{\text{entropy}}$$
$$+ \sum_{n=1}^{n=1\ldots m} (\beta_{\text{fractal}}^{n(i)} + \beta_{\text{mean}}^{n(i)} + \beta_{\text{area}}^{n(i)})$$

Where the left-hand side of equation is the natural logarithm of the odds ratio between the probabilities of a set of features belonging to and not belonging to class i. In the right-hand side, $\alpha$ denotes common texture features extracted directly from the density map ROI. Three $\beta$ are 'advanced' texture features extracted using one binary image out of a total of m different masks from an Otsu algorithm, for example, m = 5.

Thus there are 21 coefficients for one logistic model out of a total of three, corresponding to a classification in four categories. For example, with a set of known features, we can use equation (1) to calculate the possibilities of the texture features falling into class 1, 2 or 3.

The possibility of the texture features falling into class 4 is as below :

(2) $P(\text{Class} = 4) = 1 - P(\text{Class} = 1) - P(\text{Class} = 2) - P(\text{Class} = 3)$

The derived coefficient values of the regression models are summarised in Table 1 (next page). The p-values smaller than 0.05 are underlined; the model coefficients with significant (p<0.05) values are boxed.

As a parallel regression (proportional odds model) is used, the models have different intercepts but common slopes among categories. The value of the slope coefficient indicates the amount of impact of a particular feature to the odds ratio. For example, the coefficient $\alpha_{\text{kurtosis}}$ estimate of -27.31 indicates that a unit change in kurtosis, would impact the odds of an image being in a category versus not being in a category, by a factor of exp(-27.31) all else being equal.

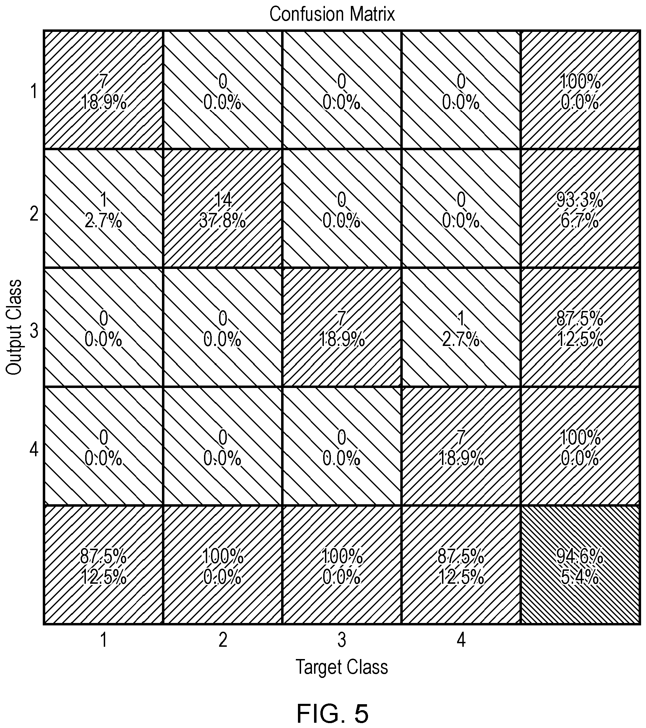

By way of example, using equation (1) with the coefficients in Table 1, the BPE categories are evaluated for 37 mammograms. The results are summarised in a confusion matrix as displayed in Figure 5.

## Table 1.   Multinomial Regression Model Coefficients

| Model coefficient Texture Feature | Model 1 | Model 2 | Model 3 | p Value | | |
|---|---|---|---|---|---|---|
| $\alpha_0$ | 690.12 | 698.23 | 701.96 | <u>0.02</u> | <u>0.02</u> | <u>0.02</u> |
| $\alpha_{mean}$ | -377.17 | -377.17 | -377.17 | 0.22 | | |
| $\alpha_{var}$ | -1068.72 | -1068.72 | -1068.72 | 0.21 | | |
| $\alpha_{skewness}$ | -64.21 | -64.21 | -64.21 | 0.12 | | |
| $\alpha_{kurtosis}$ | -27.31 | -27.31 | -27.31 | <u>0.02</u> | | |
| $\alpha_{entropy}$ | -132.89 | -132.89 | -132.89 | <u>0.01</u> | | |
| $\beta^1_{fractal}$ | 136.37 | 136.37 | 136.37 | 0.06 | | |
| $\beta^1_{mean}$ | -3.25 | -3.25 | -3.25 | <u>0.04</u> | | |
| $\beta^1_{area}$ | 0.02 | 0.02 | 0.02 | <u>0.02</u> | | |
| $\beta^2_{fractal}$ | -20.88 | -20.88 | -20.88 | 0.82 | | |
| $\beta^2_{mean}$ | -4.18 | -4.18 | -4.18 | <u>0.02</u> | | |
| $\beta^2_{area}$ | -0.02 | -0.02 | -0.02 | <u>0.02</u> | | |
| $\beta^3_{fractal}$ | 203.69 | 203.69 | 203.69 | <u>0.04</u> | | |
| $\beta^3_{mean}$ | 4.54 | 4.54 | 4.54 | <u>0.04</u> | | |
| $\beta^3_{area}$ | -0.02 | -0.02 | -0.02 | <u>0.02</u> | | |
| $\beta^4_{fractal}$ | 323.87 | 323.87 | 323.87 | 0.06 | | |
| $\beta^4_{mean}$ | 6.15 | 6.15 | 6.15 | <u>0.01</u> | | |
| $\beta^4_{area}$ | -0.02 | -0.02 | -0.02 | <u>0.02</u> | | |
| $\beta^5_{fractal}$ | -356.14 | -356.14 | -356.14 | <u>0.02</u> | | |
| $\beta^5_{mean}$ | -2.52 | -2.52 | -2.52 | 0.07 | | |
| $\beta^5_{area}$ | 0.02 | 0.02 | 0.02 | <u>0.02</u> | | |

On the confusion matrix (Figure 5), the rows correspond to the predicted class (Output Class) of BPE, and the columns show the true class (Target Class) of BPE. The diagonal cells show for how many (and what percentage) of the examples the regression model correctly estimates the classes of observations. That is, it shows what percentage of the true and predicted classes match. The off-diagonal cells show incorrect classification. The column on the far right of the plot shows the accuracy for each predicted class, while the row at the bottom of the plot shows the accuracy for each true class. The cell in the bottom right of the plot shows the overall accuracy.

In Figure 5, the diagonal cells show the number and percentage of correct classifications by the regression model. For example, seven images are correctly classified as class 1, e.g. 'Minimal'. This corresponds to 18.9% of all 37 images. Similarly, 14 cases are correctly classified as class 2, 'Mild'. This corresponds to 37.8% of all images.

One of the 'Marked' images is incorrectly classified as 'Moderate' and this corresponds to 2.7% of all 37 images in the data. Similarly, one 'Minimal' is incorrectly classified as 'Mild' and this corresponds to 2.7% of all data.

Out of seven 'Minimal' predictions, 100% are correct. Out of 15 'Mild' predictions, 93.3% are correct and 6.7% are wrong. Out of eight 'Moderate' cases, 87.5% are correctly predicted and 12.5% are predicted as 'Marked'. Out of 7 'Marked' cases, 100% are correctly classified.

Overall, 94.6% of the predictions are correct and 5.4% are incorrect classifications.

Additionally, the embodiment relates to tissue pattern complexity. Thus, in one embodiment, fractal dimension is preferably used to determine BPE category.

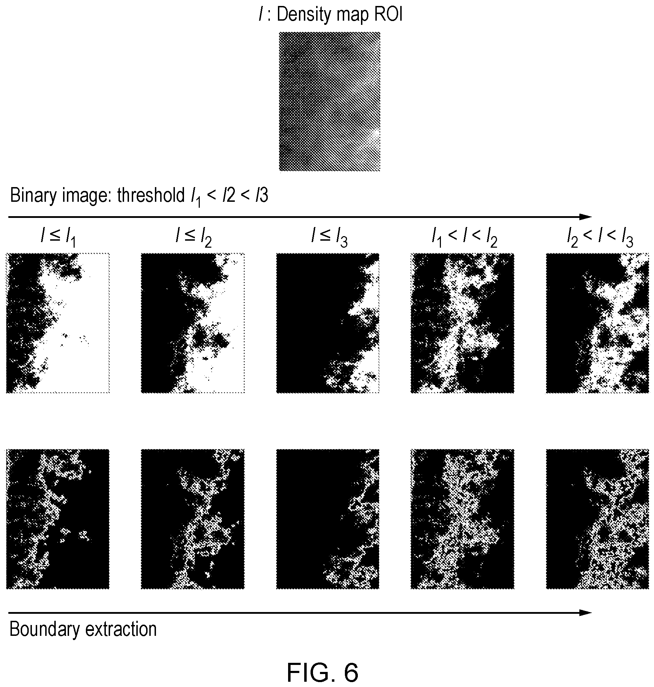

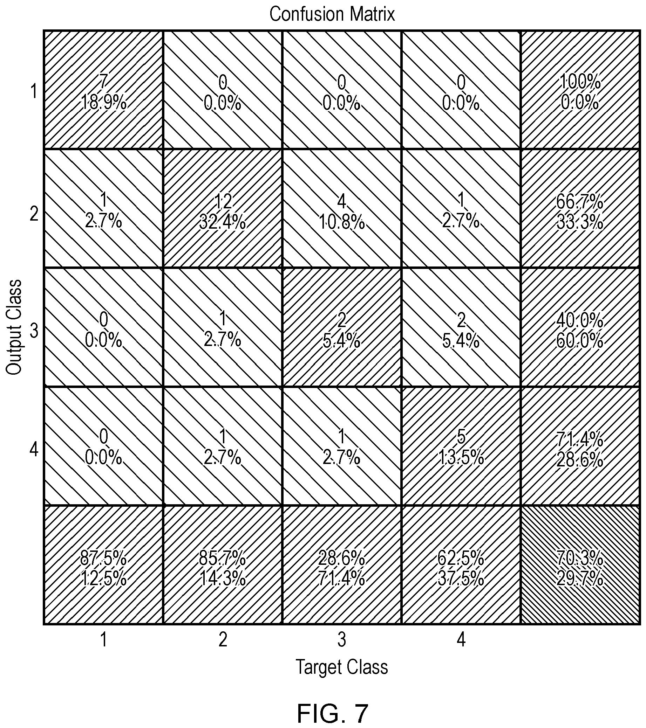

A procedure of deriving fractal dimension is illustrated in Figure 6. From a ROI image in gray-scale, three ascending thresholds are determined: $I_1 < I_2 < I_3$, thus yielding three single thresholded binary masks. Using multiple Otsu algorithm, two masks are obtained using threshold intervals $(I_1, I_2)$ and $(I_2, I_3)$. From these binary masks, their corresponding boundary maps are extracted and serve to calculate the Hausdorff fractal dimension by a simple box counting algorithm. Recalling the definition of fractal dimension, its value describes the roughness or complexity of the object boundary. As a result, BPE category may be correlated to the tissue structure complexity. That is, the more irregular pattern the tissues exhibit, the higher BPE category a breast may belong to. This is supported by the moderate prediction accuracy of 70.3% using fractal dimension only (as illustrated in Figure 7).

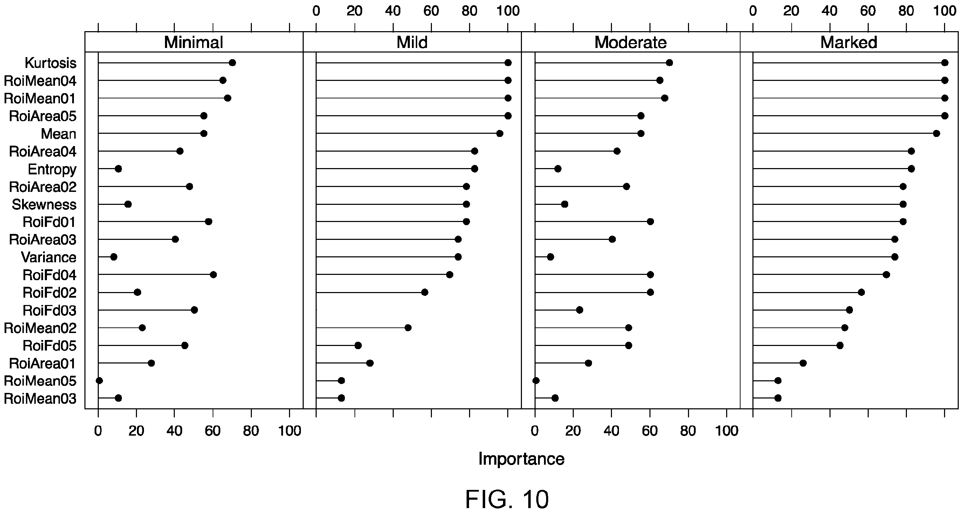

Figure 10 shows an example of two breasts: one shown in an image on the left is dense with low a BPE reading and the other shown in an image shown on the right is fatty with high BPE reading. The tissue structure of the right breast is more complicated than the tissue structure of the left breast, which further supports our understanding of a possible positive correlation between tissue pattern complexity and BPE category.

In a further embodiment to accurately and reliably determine BPE, feature selection is performed to reduce the dimension and the complexity of the modelling with more than ten observations per predictor. Feature selection methods include for example:

Backward Selection - fitting a model using all features. Then the least significant feature is dropped. A reduced model is successively re-fitted until all remaining variables are statistically significant. Four features are finally retained:

$$(3)$$

$$\beta^1_{\mathrm{mean}} \beta^3_{\mathrm{mean}} \beta^4_{\mathrm{area}} \beta^4_{\mathrm{fractal}}$$

They build a model with the accuracy of only 35.1 %.

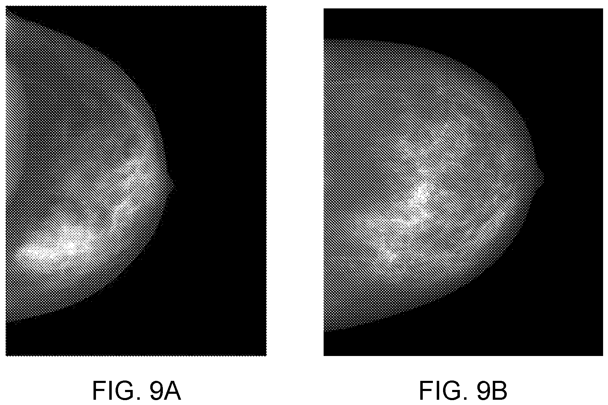

Learning Vector Quantization Model - constructed from the full feature matrix reports an importance index as an indicator for feature selection, where some features are important in one category but not in others. With reference to Figure 9 the importance of the texture features extracted from the ROI are shown for the BPE classes. Variance is the least important feature in 'Minimal' and 'Moderate' classes, but important in 'Mild' and 'Marked'.

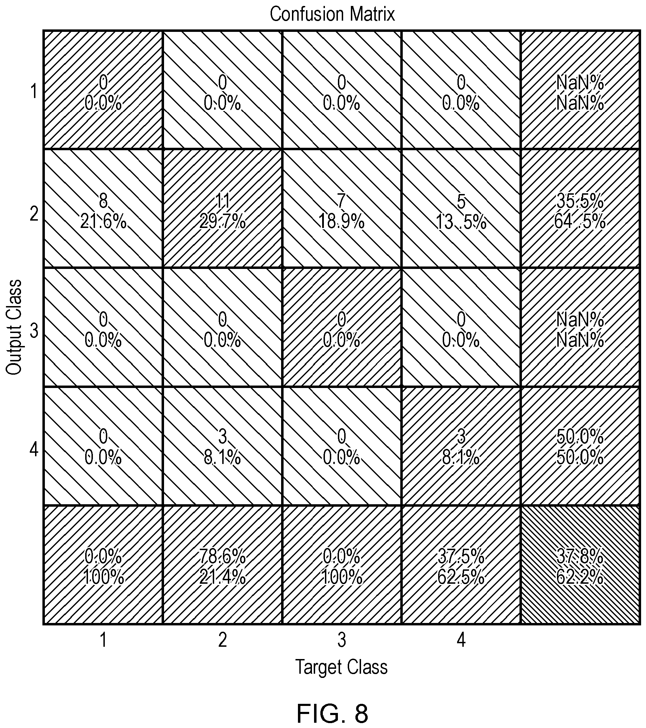

Recursive Feature Elimination - based on random forest selection function whereby, as illustrated in Figure 8, eleven attributes give the most comparable results, and three attributes give the worst results.

Other algorithms for feature selection include Boruta algorithm and Least Absolute Shrinkage and Selection Operator (LASSO).

In a further embodiment the method is applied to CAD whereby the efficacy of a CAD marker is determined by the use of the CAD marker to verify an image parameter. The image parameter, once verified, guides the determination of an object and the relevance and reliability of the marker. Among other benefits, the number of falsely identified features ('false positives') is reduced without dependence on visual perception.

CAD marks are identified on single studies (a single study might include more than one image) and the marked images are compared to stored images (patient specific stored images and/or cumulated reference images) similarly classified in order to identify 'high risk' studies by means of the image quality marks and discrepancies and the single studies are in turn stored. An illustrative example comprises the steps of:

1/ x-ray images of a breast are processed (on-site or sent to the Cloud)

2/ qualitative parameters (such as density, compression, dose, positioning, contrast-to-noise ratio and blur are computed and an image 'quality score' calculated)

3/ CAD is run on the images

4/ the CAD marks are combined with the verified parameters to ascertain an 'overall risk score'

5/ the 'overall risk score' identifies patients 'at risk' and/or at 'high risk' and alerts the reader

The 'overall risk score' comprises for example comparison of the discrepancy between the quantity and types of CAD marks; and between contemporaneous images for example between the left and right.

Further advantages include:

an overview of incidence and importance of marks to generate a 'likelihood of missing' score
a motion blur feature, especially location of motion blur: for example, motion blur is initially identified, then motion blur and related markers on the periphery is discounted to prioritise motion blur markers in locations related to risk e.g. the centre. BLUR markers are prioritised that are on or within a predetermined vicinity of features, for example, lobules in the breast, in particular lobule types II and III.

Thus, qualitative verification of CAD marks improve workflow efficiencies : informing a risk model relating to highest confidence CAD marks, patients with the 'highest likelihood of missing', and/or patient ranking based on density and/or CAD marks. Further, in addition to reducing 'false positives' the present invention helps avoid other errors: for example, where a reader might otherwise reject an image as 'not clinically acceptable' based on automated objective measures; where there are no CAD marks because an image is blurred; and where an image is optimally compressed and the image clarity causes more and 'higher confidence' CAD marks; where CAD marks appear in one part of an image and not another, indicating blur or poor compression in that part of the image rather than location of ROI.

**Claims**

1. A method for validating one or more native image parameters including thickness of a breast in compression from one or more images of the breast, wherein: one or more native parameter(s) from the image(s) is analysed with a reference data to determine whether the native parameter(s) is/are plausible, and the image(s) is/are transformed quantitatively to a tissue composition map(s), **characterised in that** an adjusted thickness of the breast is calculated for the or any of the native parameter(s) determined to be non-plausible which relies on a variable feature of the map(s) by adjusting the native parameter(s) towards plausibility and the compression based on the adjusted thickness of the breast.

2. The method according to claim 1 including forming a collective group of the native parameters and determining the plausibility of all of the native parameters in the collective group.

3. The method according to claim 1 wherein the native parameter(s) which relies on the variable feature is selected from one of the thickness of the breast or breast volume.

4. The method according to any preceding claim wherein the native parameter(s) which relies on the variable feature is/are adjusted toward plausibility based on the breast density or breast position.

5. The method according to any of claims 1 to 4 wherein the thickness of the breast is determined to be non-plausible in at least one of a plurality of the images obtained at different times; and the thickness of the breast in compression is adjusted toward plausibility by a computation in which constant breast volume is assumed.

6. The method according to claim 1 wherein a weighted image parameter is calculated by assigning a weighting to any native parameter(s) determined to be non-plausible which relies on an integral property of the breast.

7. The method according to claim 6 wherein the integral property is a foreign object, presence of cancer, predicted BPE class or breast arterial calcification.

8. The method according to claim 6 or 7 wherein based on a regression model, predicted BPE measures derived from breast tissue composition maps, determined from processing a mammogram image of the breast to generate a density map as a standardized base image, are categorised into ordinal BPE classes.

9. The method according to claim 8 wherein texture features are extracted from a region of interest in the density map and used in the regression model.

10. The method according to claim 1 wherein a weighted image parameter is calculated by assigning a weighting to any native parameter(s) determined to be non-plausible which relies on a fundamental image characteristic.

11. The method according to any preceding claim wherein image parameters are checked for whether they are used in multivariate measure and if so, the multivariate measure is re-generated using at least one adjusted image parameter.

12. The method according to claim 11 wherein input measures for multivariate measure include volumetric breast density, predicted BPE category, change in breast density over time, patient dose, breast arterial calcification scores, CAD markers, and/or risk of disease.

13. A system for validating one or more native image parameters including thickness of a breast in compression from one or more images of the breast, comprising an apparatus for validating the native image parameters, including: a device to utilize the one or more images of the source object; a device to analyse the one or more native parameters with the reference data to determine whether the native parameters are plausible; a device to transform quantitatively the image(s) to the tissue composition maps; and a device to calculate an adjusted thickness of the breast for any of the native parameter(s) determined to be non-plausible which relies on a variable feature of the map(s) by adjusting the native parameter(s) towards plausibility and the compression based on the adjusted thickness of the breast.

**Patentansprüche**

1. Verfahren zum Validieren eines oder mehrerer nativer Bildparameter, beinhaltend die Dicke einer Brust unter Kompression aus einem oder mehreren Bildern der Brust, wobei: ein oder mehrere native Parameter von dem Bild bzw. den Bildern mit einem Referenzdatensatz analysiert werden, um zu bestimmen, ob der bzw. die nativen Parameter plausibel sind, und das Bild bzw. die Bilder quantitativ in eine bzw. mehrere Gewebezusammensetzungskarten umgewandelt werden, **dadurch gekennzeichnet, dass** eine justierte Dicke der Brust für den bzw. die oder beliebige des bzw. der nativen Parameter, die als nicht plausibel bestimmt werden und die auf einem variablen Merkmal der Karte bzw. der Karten beruhen, durch Justieren des bzw. der nativen Parameter zur Plausibilität hin und der Kompression auf der Basis der justierten Dicke der Brust berechnet wird.

2. Verfahren nach Anspruch 1, beinhaltend ein Bilden einer kollektiven Gruppe der nativen Parameter und ein Bestimmen der Plausibilität aller der nativen Parameter in der kollektiven Gruppe.

3. Verfahren nach Anspruch 1, wobei der bzw. die nativen Parameter, die auf dem variablen Merkmal beruhen, aus einer bzw. einem von der Dicke der Brust oder dem Brustvolumen ausgewählt sind.

4. Verfahren nach einem vorhergehenden Anspruch, wobei der bzw. die nativen Parameter, die auf dem variablen Merkmal beruhen, auf der Basis der Brustdichte oder der Brustposition zur Plausibilität hin justiert werden.

5. Verfahren nach einem der Ansprüche 1 bis 4, wobei die Dicke der Brust in mindestens einem von einer Vielzahl von Bildern, die zu unterschiedlichen Zeiten bezogen werden, als nicht plausibel bestimmt wird; und die Dicke der Brust unter Kompression durch eine Berechnung, in der ein konstantes Brustvolumen angenommen wird, zur Plausibilität hin justiert wird.

6. Verfahren nach Anspruch 1, wobei ein gewichteter Bildparameter durch Zuweisen einer Gewichtung zu einem beliebigen bzw. mehreren beliebigen nativen Parametern, die als nicht plausibel bestimmt werden und die auf einer integralen Eigenschaft der Brust beruhen, berechnet wird.

7. Verfahren nach Anspruch 6, wobei die integrale Eigenschaft ein Fremdkörper, ein Vorliegen von Krebs, eine vorhergesagte BPE-Klasse oder eine Arterienverkalkung in der Brust ist.

8. Verfahren nach Anspruch 6 oder 7, wobei auf der Basis eines Regressionsmodells vorhergesagte BPE-Messgrößen, die von Brustgewebezusammensetzungskarten abgeleitet sind, die aus einem Verarbeiten eines Mammographiebildes der Brust bestimmt werden, um eine Dichtekarte als ein standardisiertes Basisbild zu erzeugen, in ordinale BPE-Klassen kategorisiert werden.

9. Verfahren nach Anspruch 8, wobei Texturmerkmale aus einer Region von Interesse in der Dichtekarte extrahiert und in dem Regressionsmodell verwendet werden.

10. Verfahren nach Anspruch 1, wobei ein gewichteter Bildparameter durch Zuweisen einer Gewichtung zu einem beliebigen bzw. mehreren beliebigen nativen Parametern, die als nicht plausibel bestimmt werden und die auf einem fundamentalen Bildcharakteristikum beruhen, berechnet wird.

11. Verfahren nach einem vorhergehenden Anspruch, wobei Bildparameter darauf geprüft werden, ob sie in einer multivariaten Messgröße verwendet werden, und wenn dies der Fall ist, die multivariate Messgröße unter Verwendung mindestens eines justierten Bildparameters regeneriert wird.

12. Verfahren nach Anspruch 11, wobei eingegebene Messgrößen für eine multivariate Messgröße beinhalten die volumetrische Brustdichte, die vorhergesagte BPE-Kategorie, die Veränderung der Brustdichte im Zeitablauf, die Patientendosis, die Scores einer Arterienverkalkung in der Brust, die CAD-Marker und/oder das Krankheitsrisiko beinhalten.

13. System zum Validieren eines oder mehrerer nativer Bildparameter, beinhaltend die Dicke einer Brust unter Kompression aus einem oder mehreren Bildern der Brust, umfassend eine Ausrüstung zum Validieren der nativen Bildparameter, beinhaltend: eine Vorrichtung zum Nutzen des einen oder der mehreren Bilder des Quellobjekts; eine Vorrichtung zum Analysieren des einen oder der mehreren nativen Parameter mit den Referenzdaten, um zu bestimmen, ob die nativen Parameter plausibel sind; eine Vorrichtung zum quantitativen Umwandeln des Bildes bzw. der Bilder in die Gewebezusammensetzungskarten und eine Vorrichtung zum Berechnen einer justierten Dicke der Brust für beliebige des bzw. der nativen Parameter, die als nicht plausibel bestimmt werden und die auf einem variablen Merkmal der Karte bzw. der Karten beruhen, durch Justieren des bzw. der nativen Parameter zur Plausibilität hin und der Kompression auf der Basis der justierten Dicke der Brust.

**Revendications**

1. Un procédé de validation d'un ou plusieurs paramètres d'image natifs incluant l'épaisseur d'un sein en compression à partir d'une ou plusieurs images du sein, dans lequel : un ou plusieurs paramètre(s) natif(s) des image(s) est/sont analysé(s) avec des données de référence pour déterminer si le ou les paramètre(s) natif(s) est/sont plausible(s), et la ou les image(s) est/sont transformée(s) quantitativement en carte(s) de composition de tissu, **caractérisé en ce qu'**une épaisseur du sein ajustée est calculée pour l'un quelconque du ou des paramètre(s) natif(s) déterminé(s) ne pas être plausible(s) dépendant d'une fonction variable de la ou des carte(s) en ajustant le ou les paramètre(s) natif(s) vers la plausibilité et la compression sur la base de l'épaisseur du sein ajustée.

2. Le procédé selon la revendication 1 incluant la formation d'un groupe collectif des paramètres natifs et la détermination de la plausibilité de tous les paramètres natifs dans le groupe collectif.

3. Le procédé selon la revendication 1, dans lequel le ou les paramètre(s) natif(s) dépendant de la fonction variable est/sont sélectionné(s) parmi soit l'épaisseur du sein, soit le volume du sein.

4. Le procédé selon l'une quelconque des revendications précédentes, dans lequel le ou les paramètre(s) natif(s) dépendant de la fonction variable est/sont ajusté(s) vers la plausibilité sur la base de la densité du sein ou de la position du sein.

5. Le procédé selon l'une quelconque des revendications 1 à 4, dans lequel l'épaisseur du sein est déterminée ne pas être plausible dans une ou plusieurs image(s) d'une pluralité d'images obtenues à différents moments ; et l'épaisseur du sein en compression est ajustée vers la plausibilité par un calcul dans lequel un volume constant du sein est supposé.

6. Le procédé selon la revendication 1, dans lequel un paramètre d'image pondéré est calculé en attribuant une pondération à l'un quelconque des paramètre(s) natif(s) déterminé(s) ne pas être plausible(s) dépendant d'une propriété intégrale du sein.

7. Le procédé selon la revendication 6, dans lequel la propriété intégrale est un objet étranger, la présence de cancer, la classe BPE prédite ou la calcification artérielle du sein.

8. Le procédé selon la revendication 6 ou 7, dans lequel sur la base d'un modèle de régression, des mesures BPE prédites, dérivées de cartes de composition du tissu mammaire, déterminées par le traitement d'une image de mammographie du sein pour générer une carte de densité en tant qu'image de base standardisée, sont classées dans des classes BPE ordinales.

9. Le procédé selon la revendication 8, dans lequel les fonctions de texture sont extraites d'une région d'intérêt dans la carte de densité et utilisées dans le modèle de régression.

10. Le procédé selon la revendication 1, dans lequel un paramètre d'image pondéré est calculé en attribuant une pondération à un ou plusieurs paramètre(s) natif(s) déterminé(s) n'étant pas plausible(s) dépendant d'une caractéristique d'image fondamentale.

11. Le procédé selon l'une quelconque des revendications précédentes, dans lequel des paramètres d'image sont vérifiés pour savoir s'ils sont utilisés dans des mesures multivariées et, le cas échéant, les mesures multivariées sont générées à nouveau en utilisant un ou plusieurs paramètre(s) d'image ajusté(s).

12. Le procédé selon la revendication 11, dans lequel des mesures d'entrée pour des mesures multivariées incluent la densité volumétrique du sein, la catégorie BPE prédite, le changement de densité du sein dans le temps, la dose administrée au patient, les scores de calcification artérielle du sein, les marqueurs CAD et/ou le risque de maladie.

13. Un système de validation d'un ou plusieurs paramètres d'image natifs incluant l'épaisseur d'un sein en compression à partir d'une ou plusieurs images du sein, comprenant un appareil de validation des paramètres d'image natifs, incluant : un dispositif pour utiliser la ou les images de l'objet source ; un dispositif pour analyser le ou les paramètres natifs avec les données de référence pour déterminer si les paramètres natifs sont plausibles ; un dispositif pour transformer quantitativement la ou les image(s) sur les cartes de composition de tissu ; et un dispositif pour calculer une épaisseur du sein ajustée pour l'un quelconque du ou des paramètre(s) natif(s) déterminé(s) n'étant pas plausible(s) dépendant d'une fonction variable de la ou des carte(s) en ajustant le ou les paramètre(s) natif(s) vers la plausibilité et la compression sur la base de l'épaisseur du sein ajustée.

FIG. 1

Time T

FIG. 2A

Time T + I

FIG. 2B

Time T

FIG. 3A

Time T + I

FIG. 3B

(a)

FIG. 4A

(b)

FIG. 4B

(c)

FIG. 4C

(d)

FIG. 4D

Confusion Matrix

FIG. 5

*I* : Density map ROI

Binary image: threshold $I_1 < I2 < I3$

$I \leq I_1$     $I \leq I_2$     $I \leq I_3$     $I_1 < I < I_2$     $I_2 < I < I_3$

Boundary extraction

FIG. 6

Confusion Matrix

FIG. 7

Confusion Matrix

FIG. 8

FIG. 9A

FIG. 9B

FIG. 10

FIG. 11

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 8582840 B2
- GB 2010OO1472 W
- WO IB2017054382 A

### Non-patent literature cited in the description

- **HIGHNAM et al.** *Impact of errors in recorded compressed breast thickness measurements on volumetric density classification,* 2016
- **NG ; LAU.** *Vision 20/20: Mammographic breast density and its clinical applications,* 2015
- **HIGHNAM ; BRADY.** *Mammographic Image Analysis,* 1999